# EUROPEAN PATENT SPECIFICATION

(11) **EP 1 044 036 B1**
(45) Date of publication and mention of the grant of the patent: **23.06.2004**
(21) Application number: 97952697.7
(22) Date of filing: 29.12.1997
(51) Int. Cl.: A61N 2/02

(54) **TREATMENT DEVICE FOR LUMBAR AND COXOFEMORAL ORGANS**
VORRICHTUNG ZUR BEHANDLUNG VON LENDEN- UND HÜFTENOBERSCHENKEL-ORGANEN
DISPOSITIF SERVANT A TRAITER LES ORGANES LOMBAIRES ET COXO-FEMORAUX

(43) Date of publication of application: 18.10.2000
(73) Proprietor: Pletnev, Sergey Vladimirovich, Minsk, 220070 (BY)
(72) Inventor: Pletnev, Sergey Vladimirovich, Minsk, 220070 (BY)
(74) Representative: Viering, Jentschura & Partner
(86) International application number: PCT/BY1997/000010
(87) International publication number: WO 1999/033519

(56) References cited:
- EP-A- 0 788 813
- WO-A-91/15180
- WO-A-96/36401
- DE-U- 9 300 499
- US-A- 5 123 916
- US-A- 5 269 738

## Description

The present invention relates to the field of medicine and in particular to prophylaxis and therapy of diseases of lumbar and coxofemoral organs and the prostate of a human body.

The prior art DE 93 00 499 Ul discloses a furniture for seating comprising a magnetotherapy device with at least one coil. Further, devices for training muscles of locomotor system of a human body, rehabilitation after various diseases, prophylaxis of diseases are known in the prior art. The devices comprise a supporting frame with a seat and dynamic elements for physical work of muscles. For better effect the devices are provided with an impedance generator allowing to control the individual physical work of each patient.

The trainer may have various building composition, different types of impedance generators and methods of their control / 1, 2, 3, 4, 5, 6 /.

Prior art teaches on use of a therapeutic effect of magnetic field for prophylaxis and treatment of various organs of a human body. The prior devices for magnetotherapy produce favorable effect both on the whole human body and on its selected organs /7, 8, 9/. In particular, devices for pulse magnetotherapy of gynecological diseases, prostatitis, etc., use pulse magnetic fields located in the immediate vicinity to treated organs /10, 11 /. The devices are generally used at clinics and require constant control of medical personnel.

The claimed invention provides a device for dynamic physical work of the locomotor system of a human body. The seat of the device is mechanically connected to the dynamic elements for physical work of muscles. The seat further comprises elements for carrying out magnetotherapy of lumbar and coxofemoral organs and the prostate of a human body. The elements comprise a magnetic inductor, integral within the seat, and an impulse generator connected with the magnetic inductor through a modulating device.

The device additionally comprises a motion detector of the dynamic element. Physical work of a patient combined with the magnetic field effecting the body organs improves circulation of the blood, metabolism, hormonal processes, rehabilitation activity of physiological functions. A seats position of a patient rotating (pressing) the dynamic elements produces alternating compression and displacement of lumbar and coxofemoral organs of a human body making thereby massage of the organs. At the moment the motion detector generates a signal and supplies it to the modulating device. Accordingly, the effect of the magnetic field is synchronised with the location of an organ which is more favourable for creating an effect. Certain position of the locomotor system at maximum extension of muscular tissue provide the best approach of internal organs and the inductor, in particular of the prostate and the inductor.

According to one embodiment of the invention the device is additionally equipped with a audiosystem for listening-in music. Synchronously with the physical work on the trainer a patient is listening to harmonious musical sounds of the frequency that stimulates natural functions of a certain human organ. In parallel to that a signal from the audiosystem is supplied to the modulating device. Thereby the magnetic field is synchronized with the musical sound vibration effecting the human organ during listening-in.

The use of the aforementioned elements eliminates the necessity to introduce the inductor into the body and affords to use the magnetotherpy method and device outside a clinic.

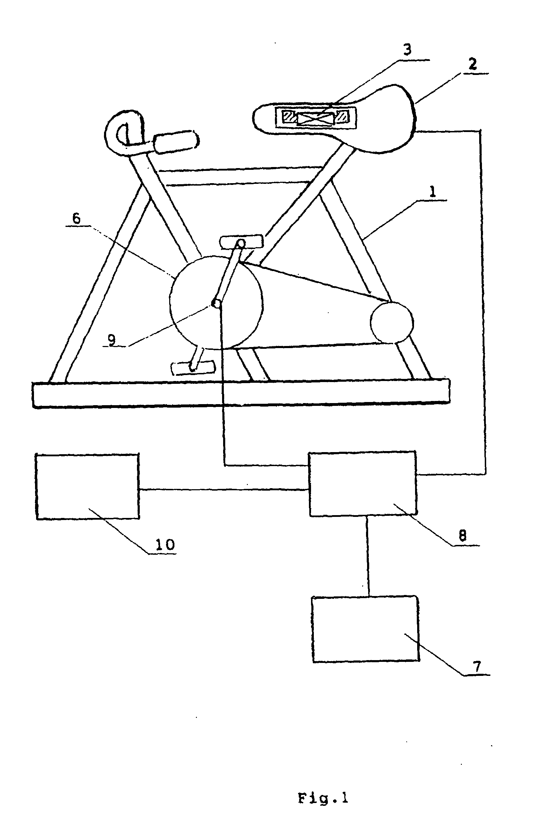

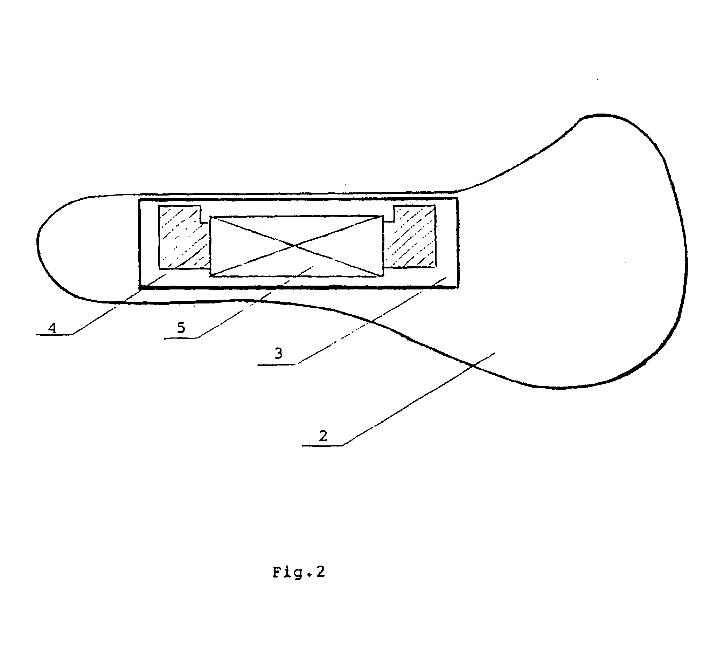

A bicycle trainer serves an example of an embodiment of the invention. Figure 1 is a diagram of the basic principle for carrying out the invention regarding magnetotherapy of a prostate. The claimed device for prophylaxis and treatment of lumbar and coxofemoral organs of a human body comprises a supporting frame having a base 1, a seat 2, dynamic elements 6, magnetic inductor 3 located in the seat 2, pulse generator 7 connected with the inductor 3 through the modulating device 8. Figure 2 is a general view of the seat 2 of the bicycle trainer. The seat 2 includes the inductor 3 located inside it and comprising a magnetic conductor 4 and an electric coil 5 producing a local magnetic field. Location of the inductor 3 in the seat 2 provides maximum approach of the inductor to the lumbar and coxofemoral organs of a human body.

The inductor 3 is pointed to provide maximum approach to the prostate and to locate and concentrate the magnetic field in the immediate vicinity to the prostate during the physical work.

A signal from the pulse generator 7 is supplied to the electric coil 5 of the inductor 3 through the modulating device 8 forming a magnetic field of specified characteristics. In one of the embodiments the inductor 3 may be a set of electromagnetic elements, then the modulating device 8 operates as a switching device as well.

The trainer is additionally provided with a detector of motion of a dynamic element 9 connected with the control input of the modulating device 8. The said embodiment of the claimed device allows to synchronise the magnetic field modulation with rotating (pressing) of dynamic elements registering thereby the point of the best approach of the organ with the inductor 3. During rotation of dynamic elements 6, points of magnetic field modulation are selected so that to agree them with the points of the maximum strain of the muscles of the lumbar and coxofemoral organs of a human body and with approach of the prostate to the inductor 3, intensifying thereby effectiveness of the prostate massage.

Exercises on trainers may be also accompanied by a stimulating sound effect. Prior art describes selective effect on functional organ activity of a tone quality of musical instruments / 12/. Prior art also discloses sound vibration effect on certain organs / 13/. Further development of the claimed system is modulation of a magnetic field with a sound (harmonic) signal by supplying it from the output of the audiosystem 10 to the modulating device 8. The said embodiment of the device allows use of musical therapy in combination with magnetic therapy. Concurrent effecting of a harmonic sound and a magnetic field modulation intensify favourable effect of the device in use.

Thus, the claimed device allows to control the therapeutic treatment of human organs at lower characterics of magnetic field and to stimulate natural organ functions producing thereby greater treating effect.

The claimed device fabricated on the basis of a bicycle trainer was tested in a clinic and showed good results in prophylaxis and treatment of prostatitis. The device may be used for prophylaxis and treatment of diseases of lumbar and coxofemoral organs of a human body in sanatoria, trainer halls, at home, during long term human stay in a bounded space ( e.g. in submariners, spaceships) allowing to disengage medical staff.

### Sources of information

1. Exercise bicycle. **US** Nº 5397286, A63 B 21/00.
2. Centrifugal resistance device for stationary bicycle trainer. **US** Nº5397285, A63 B 21/22.
3. Recumbent total body exerciser **PCT** patent appln. No. 9427678, A63 B 21/00.
4. Pneumatic variable resistance rehabilitation/therapy apparatus **US** Nº5312315, A 63 B21/008.
5. Riding exerciser. **US** Nº5356357, A63 B 21/00, 69/06.
6. Self-powered human centrifuge **US** Nº5378214, A63 B 21/00.
7. Chair having an inductor embedded within the seat, further comprising an inductor. DE-U-9300499, A 47 C 9/02.
8. Physiotherapy Manual (under edition of V.G. Jasnogorodski), M., Medicina, 1992, p.13.
9. Methodical Reference Book ( Differential Use of Magnetic Fields for Neurologic Phenomena of Lumbar Octeochondrosis. NIINNF of the Ministry of Health of the Republic of Belarus, Minsk, 1990), p.14.
10.Method of pulse magnetotherapy of biological objects and device therefore. RU94009616, A61 N 2/00
11.Device for magnetotherapy **BY** Nº950918, A61 N2/00.
12.2^{nd} International Conference " Theoretical and clinical aspects of bioresonance and multiresonace therapy. Abstracts and reports. IMRDIZ editor, Moscow, 1996, p.21-25.
13. 2^{nd} International Conference " Theoretical and clinical aspects of bioresonance and multiresonance therapy. Abstracts and reports. IMRDIZ editor, Moscow, 1996, p.25-30.

## Claims

1. A device for prophylaxis of diseases of lumbar and coxofemoral organs and the prostate of a human body comprising a seat (2) which comprises a magnetic inductor (3) and a pulse generator (7) connected to the magnetic inductor (3), **characterised in that** the device comprises dynamic elements (6) for physical work of muscles that are mechanically connected to the seat (2) and the magnetic inductor (3) is connected to the pulse generator (7) through a modulating device (8).

2. A device according to claim 1, **characterised in that** it comprises a detector of motion (9) of a dynamic element (6) connected with the control input of the modulating device (8).

3. A device according to claim 1, **characterised in that** it further comprises an audiosystem (10) for listening-in music, the output of which is connected to the modulating device (8).

## Patentansprüche

1. Vorrichtung zur Prophylaxe von Krankheiten lumbaler und koxofemoraler Organe und der Prostata eines menschlichen Körpers, das einen Sitz (2) aufweist, der einen magnetischen Induktor (3) und einen Pulsgenerator (7) aufweist, der mit dem magnetischen Induktor (3) gekoppelt ist, **dadurch gekennzeichnet, dass** die Vorrichtung dynamische Elemente (6) für physische Arbeit von Muskeln aufweist, welche dynamischen Elemente (6) mechanisch mit dem Sitz gekoppelt sind und der magnetische Induktor (3) mittels einer Modulationsvorrichtung (8) mit dem Pulsgenerator (7) gekoppelt ist.

2. Vorrichtung gemäß Anspruch 1, **dadurch gekennzeichnet, dass** sie einen Bewegungsdetektor (9) eines dynamischen Elements (6) aufweist, der mit dem Steuerungseingang der Modulationsvorrichtung (8) gekoppelt ist.

3. Vorrichtung gemäß Anspruch 1, **dadurch gekennzeichnet, dass** sie ferner ein Audiosystem (10) zum Hören von Musik aufweist, dessen Ausgang mit der Modulationsvorrichtung (8) gekoppelt ist.

## Revendications

1. Dispositif de prophylaxie des maladies des organes lombaires et coxo-fémoraux et de la prostate d'un corps humain, comprenant un siège (2) qui comprend un inducteur magnétique (3) et un générateur d'impulsions (7) connecté à l'inducteur magnétique (3), **caractérisé en ce que** le dispositif comprend des éléments dynamiques (6) permettant le travail physique des muscles qui sont connectés mécaniquement au siège (2) et l'inducteur magnétique (3) est connecté au générateur d'impulsions (7), par l'intermédiaire d'un dispositif de modulation (8).

2. Dispositif selon la revendication 1, **caractérisé en ce qu'**il comprend un détecteur de mouvement (9) d'un élément dynamique (6) connecté à l'entrée de commande du dispositif de modulation (8).

3. Dispositif selon la revendication 1, **caractérisé en ce qu'**il comprend en outre un système à audiofréquences (10) permettant d'écouter de la musique, dont la sortie est connectée au dispositif de modulation (8).
